(19) Europäisches Patentamt
European Patent Office
Office européen des brevets

(11) Veröffentlichungsnummer: **0 272 201 B1**

# (12) EUROPÄISCHE PATENTSCHRIFT

(45) Veröffentlichungstag der Patentschrift: **08.07.92**

(51) Int. Cl.5: **C12P 41/00**, C12P 17/02, C12P 7/62, C07C 405/00

(21) Anmeldenummer: **87730145.7**

(22) Anmeldetag: **12.11.87**

## (54) Stereospezifische Ketoreduktion von Bicyclooctandion-carbonsäureestern durch Mikroorganismen.

(30) Priorität: **13.11.86 DE 3638759**

(43) Veröffentlichungstag der Anmeldung:
**22.06.88 Patentblatt 88/25**

(45) Bekanntmachung des Hinweises auf die Patenterteilung:
**08.07.92 Patentblatt 92/28**

(84) Benannte Vertragsstaaten:
**AT BE CH DE ES FR GB GR IT LI LU NL SE**

(56) Entgegenhaltungen:
**FR-A- 2 582 648**

**TETRAHEDRON, Band 42, Nr. 1, 1986, Seiten 435-444, Pergamon Press Ltd, GB; K. MORI et al.: "A new synthesis of (+)-6a-carbaprostaglandin I2 employing yeast reduction of a beta-keto ester derived from cis-bicyclo[3.3.0.]octane-3,7-dione as the key-step"**

**CHEM. PHARM. BULL., Band 33, Nr. 7, 1985, Seiten 2688-2696; K. KOJIMA et al.: "A convenient synthesis of (+)-9(0)-methanoprostacyclin"**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Petzoldt, Karl, Dr.**
**Flachsweg 10**
**W-1000 Berlin 38(DE)**
Erfinder: **Dahl, Helmut, Dr.**
**Gollanczstrasse 102**
**W-1000 Berlin 28(DE)**
Erfinder: **Vorbrüggen, Helmut, Prof.**
**Wilkestrasse 7**
**W-1000 Berlin 27(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein mikrobiologisches Verfahren zur stereospezifischen Mono-Ketoreduktion von racemischen bicyclischen Carbacyclin-Zwischenstufen der Formel (±) - II zu den entsprechenden optisch aktiven 3α-Hydroxy-Verbindungen der Formel (+) - I.

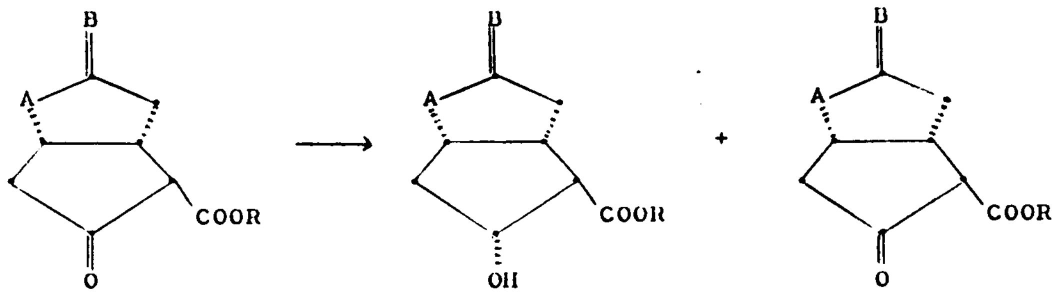

(±) - II (+) - I (-) - II

Die Reste A, B und R in den Formeln bedeuten:

A: $CH_2$ oder CH-COOR,

B: Sauerstoff,

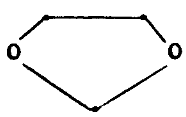
,
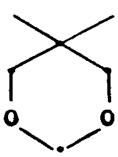

oder weitere Ketalreste und

R: $C_1$-$C_6$-Alkyl.

Das Verfahren ist dadurch gekennzeichnet, daß man ein Keton der allgemeinen Formel (±) - II, worin A, B und R die oben angegebenen Bedeutungen haben, mit Mikroorganismen behandelt und das dabei entstehende 3α-Hydroxy-prostacyclin-Zwischenprodukt, das in seiner Konfiguration dem natürlichen $PGI_2$ entspricht, isoliert.

In einer zweiten Ausführungsform des erfindungsgemäßen stereospezifischen Ketoreduktions-Verfahrens wird nicht das dem natürlichen $PGI_2$ entsprechend konfigurierte Enantiomere des racemischen Synthons (±) - II durch die Mikroorganismen reduziert, sondern die Antipodenform. In diesem Falle wird das bei der mikrobiologischen Transformation unverändert gebliebene, natürlich konfigurierte Keto-Enantiomere (+) - II für die Synthese von Prostacyclinen herangezogen, indem man es entweder chemisch (zum Beispiel mit Natriumborhydrid) oder mikrobiologisch zu (+) - I reduziert.

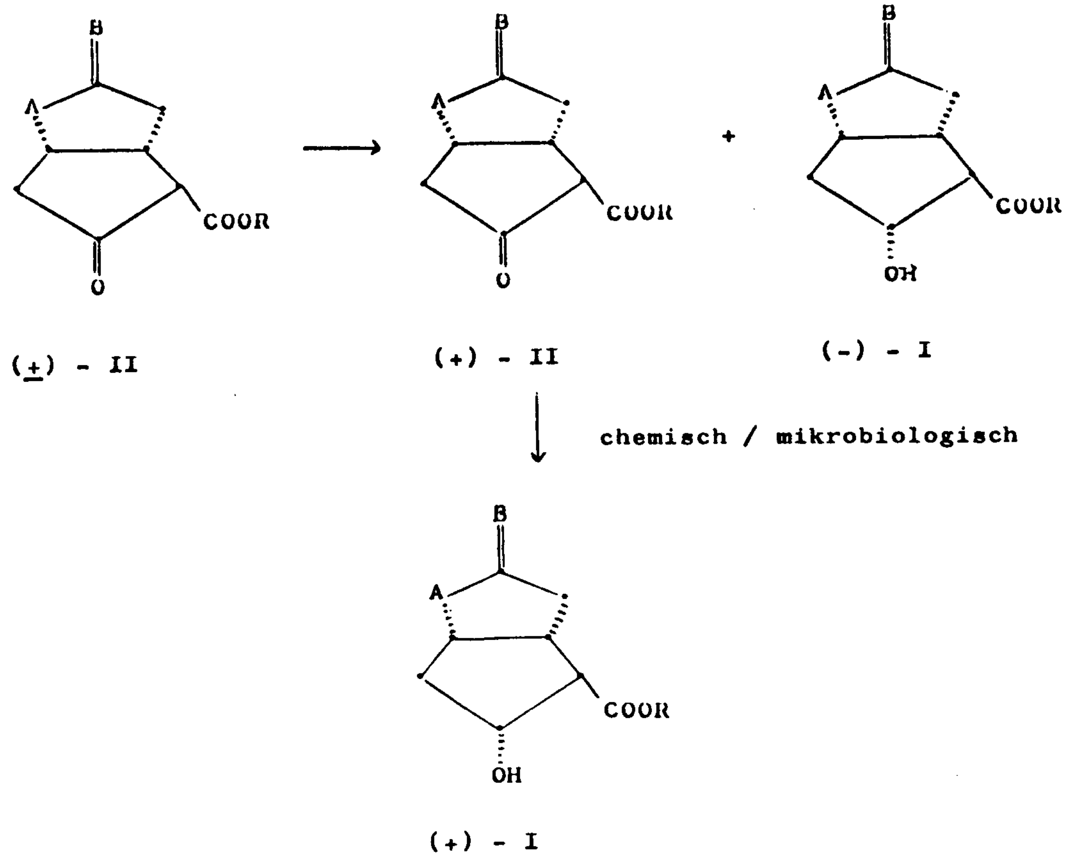


(±) - II

(+) - II

(-) - I

(+) - I

Die Erfindung eignet sich besonders zur mikrobiologischen stereospezifischen Reduktion der nachstehend aufgeführten Prostacyclin-Zwischenstufen 3 - 7.

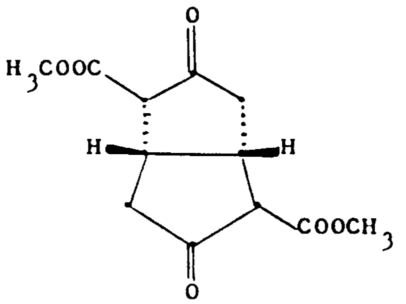

(±) - 3

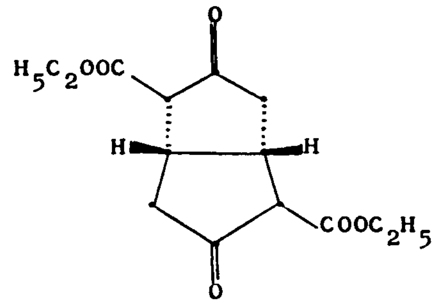

(±) - 4

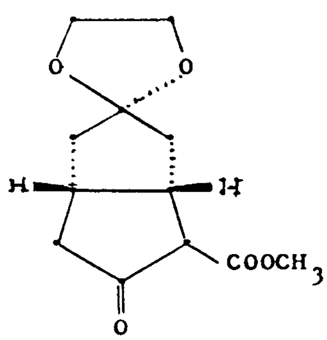

(±) - 5

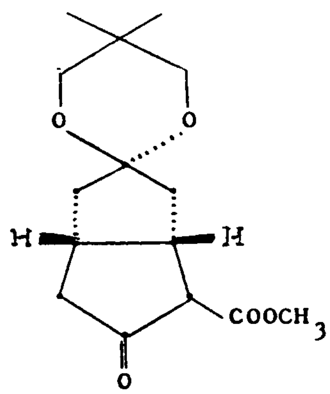

(±) - 6

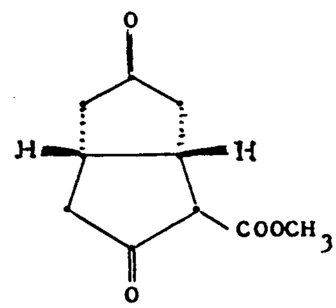

(±) - 7

Optisch aktives 6a-Carba-prostacyclin und besonders einige davon abgeleitete Verbindungen besitzen als stabile Analoge des natürlichen Prostacyclins ($PGI_2$) einen hohen therapeutischen Nutzen [R.C. Nickolson, M.H. Town, H. Vorbrüggen: Prostacyclin-Analogs, Medicinal Research Reviews, Vol. 5, No. 1, S. 1-53 (1985)]. Die in dieser neueren Übersicht aufgeführten Synthesen sind lang und führen teilweise nur zu racemischen Carbacyclinen. Besonders aufwendig sind die Synthesen, die zu Carbacyclinen in der dem natürlichen $PGI_2$ entsprechenden absoluten Konfiguration führen. Das liegt daran, daß gut zugängliche, geeignete Ausgangsmaterialien achiral sind und die optische Aktivität erst im Syntheseverlauf in hierfür geeignete Zwischenstufen eingeführt werden muß.

Mehrere Synthesen gehen bereits von optisch aktiven 7α-Hydroxy-6β-hydroxymethyl-2-oxa-bicyclo-[3.3.0]octan-3-on-Derivaten aus. Damit ist zwar das Problem der Einführung der optischen Aktivität gelöst. Es müssen jedoch noch weitere vielstufige Synthesesequenzen für die Substitution der 2-Oxa-Funktion durch eine Methylengruppe durchgeführt werden, um zu Derivaten des 3α-Hydroxy-2β-hydroxymethyl-bicyclo[3.3.0]octan-7-ons zu gelangen, die sich erst für den Anbau der für die Carbacyclin-Analoga jeweils typischen α- und ω-Ketten eignen.

Eine neuere Publikation beschreibt die Verwendung von cis-Bicyclo-[3.3.0]octan-3,7-dion-Derivaten zur Synthese optisch aktiver Carbacycline. Kojima et al. beschreiben in Chem. Pharm. Bull. 33, 2688 (1985) ein Verfahren, das die Trennung diastereomerer Salze der racemischen 7,7-Ethylendioxy-3α-hydroxy-cis-bicyclo[3.3.0]octan-2-carbonsäure einschließt.

Auch dieses Verfahren erfordert noch 7 Reaktionsschritte, um - ausgehend von 3-Oxoglutarestern - zum Startmaterial für Carbacyclin-Analoga zu gelangen. Zudem wird eine instabile β-Ketosäure-Zwischenstufe durchlaufen.

Für die Herstellung optisch aktiver Carbacyclin-Analoga, wie sie oben beschrieben werden, ist weiterhin kein Syntheseweg, der eine einfache Herstellung gestattet, bekannt.

Die erfindungsgemäße mikrobiologische Reduktion an den vorstehend aufgeführten Prostacyclin-Zwischenprodukten wird mit folgenden Mikroorganismen-Stämmen oder den daraus isolierten Enzymen durchgeführt:
Rhizopus oryzae (CBS 32947)
Rhizopus arrhizus (ATCC 34102)
Rhizopus arrhizus (ATCC 10260)
Rhodotorula spec. (ATCC 18101)
Rhodotorula mucilaginosa (NCYC 63)
Rhodotorula glutinis (ATCC 2527)
Rhizopus stolonifer (ATCC 6227 b)
Unter den verschiedenen Arten der angegebenen Klassen von Mikroorganismen treten naturgemäß Unterschiede der Wirksamkeit in der erfindungsgemäßen Ketoreduktion auf.

Am 11.11.1986 wurden die Stämme Rhizopus oryzae (DSM 3899) und Rhizopus arrhizus (DSM 3898) bei der Deutschen Sammlung von Mikroorganismen hinterlegt.

Aus den nach dem erfindungsgemäßen Verfahren hergestellten optisch aktiven 3α-Hydroxy-Verbindungen der allgemeinen Formel (+) - I lassen sich pharmakologisch wirksame Prostacycline herstellen. Beispielsweise gelangt man ausgehend von (+) - I zum Wirkstoff Iloprost (beschrieben in EP 11591).

Die Erfindung betrifft somit ein Verfahren zur Herstellung von 3α-Hydroxy-prostacyclin-Zwischenprodukten der Formel (+) - I

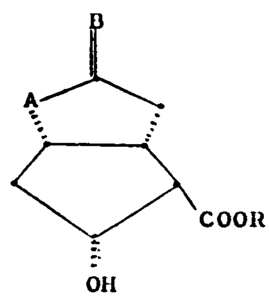

(+) - I

worin

A die Reste $-CH_2-$ oder >CH-COOR,

B Sauerstoff oder den 2-bindigen Rest -O-X-O- mit X als geradkettigem oder verzweigtkettigem Alkylen mit 1 - 7 C-Atomen und

R eine geradkettige oder verzweigtkettige Alkylgruppe mit 1 - 6 C-Atomen oder Benzyl bedeuten,

dadurch gekennzeichnet, daß man ein racemisches Bicyclooctandion der Formel (±) - II

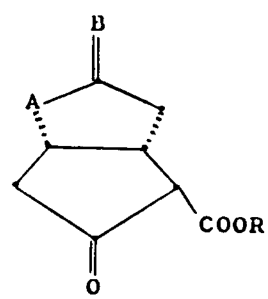

(±) - II

worin A, B und R die oben angegebene Bedeutung haben, mit Rhizopus- oder Rhodotorula- Stämmen behandelt und für den Fall, daß der eingesetzte Stamm die (-)-Form aus dem Racemat (±) - II reduziert, eine chemische oder mikrobiologische Reduktion des unveränderten Bicyclooctandions der Formel (+) - II anschließt.

Wenn X einen geradkettigen oder verzweigtkettigen Alkylenrest mit 1 - 7 C-Atomen bedeutet, so sind

damit folgende Reste gemeint:
$-(CH_2)_n-$ mit n = 1 - 7 (Methylen, Ethylen, Tri-, Tetra-, Penta-, Hexa-und Heptamethylen), $-C(CH_3)_2-$, $-CH(CH_3)-$, $CH(CH_3)-CH_2-$, $-C(CH_3)_2-CH_2-$, $-CH_2-CH(CH_3)-$, $CH_2-C(CH_3)_2-$, $-CH_2-CH(CH_3)-CH_2-$, $-CH_2-C(CH_3)_2-CH_2-$, $-CH(C_2H_5)-$, $-C(C_2H_5)_2-$, $-CH(C_2H_5)-CH_2-$, $-C(C_2H_5)_2-CH_2-$, $-CH_2-CH(C_2H_5)-$, $-CH_2-C(C_2H_5)_2-$, $-CH_2-CH(C_2H_5)-CH_2-$, $-CH_2-C(C_2H_5)_2-$ usw.

Unter R als $C_1$-$C_6$-Alkyl werden Methyl, Ethyl, n-Propyl, Isopropyl, n-Butyl, Isobutyl, sek.-Butyl, tert.-Butyl, n-Pentyl, Isopentyl, sek.-Pentyl, Neopentyl, n-Hexyl, Isohexyl usw. verstanden. Je nach der letztlich gewünschten Bedeutung von B können die Schutzgruppen nach an sich bekannten Methoden abgespalten werden.

Zunächst werden unter den für die genannten Mikroorganismen üblicherweise verwendeten Kulturbedingungen in einem geeigneten Nährmedium und unter Belüften Submerskulturen angezüchtet. Dann setzt man den Kulturen das Substrat (in einem geeigneten Lösungsmittel gelöst oder vorzugsweise in emulgierter Form) zu und fermentiert, bis eine maximale Substratumwandlung erreicht ist.

Geeignete Substratlösungsmittel sind beispielsweise Methanol, Ethanol, Glykolmonomethyläther, Dimethylformamid oder Dimethylsulfoxyd. Die Emulgierung des Substrats kann beispielsweise bewirkt werden, indem man dieses in mikronisierter Form oder in einem mit Wasser mischbaren Lösungsmittel (wie Methanol, Ethanol, Aceton, Glykolmonomethyläther, Dimethylformamid, Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Ethylenoxydaddukte oder Fettsäureester von Polyglykolen. Als geeignete Emulgatoren seien die handelsüblichen Netzmittel Tegin®, Tagat® und Span® beispielsweise genannt.

Oft ermöglicht die Emulgierung der Substrate einen erhöhten Substratdurchsatz und somit eine Steigerung der Substratkonzentration. Es ist aber selbstverständlich auch möglich, bei dem erfindungsgemäßen Verfahren andere Methoden zur Steigerung des Substratdurchsatzes, wie sie dem Fermentationsfachmann wohl bekannt sind, anzuwenden.

Die optimale Substratkonzentration, Substratzugabezeit und Fermentationsdauer ist von der Struktur des verwendeten Substrates und der Art des verwendeten Mikroorganismus abhängig. Diese Größen müssen, wie dies bei mikrobiologischen Umwandlungen allgemein erforderlich ist, im Einzelfall durch Vorversuche, wie sie dem Fachmann geläufig sind, ermittelt werden.

## Herstellung der Ausgangsverbindungen

cis-Bicyclo[3.3.0]octan-3,7-dion-2,6-dicarbonsäureester

Der Dimethylester 3 ist literaturbekannt [Weiss et al., J. Org. Chem42, 3089 (1977)] und ausgehend von 3-Oxoglutarsäuredimethylester über cis-Bicycl[3.3.0]octan-3,7-dion-2,4,6,8-tetracarbonsäure-tetramethylester in zwei Reaktionsschritten gut zugänglich:
Der Diethylester 4 läßt sich aus cis-Bicyclo[3.3.0]octan-3,7-dion-2,4,6,8-tetracarbonsäure-tetraethylester (EP 33863) herstellen.

Höhere Ester können analog zu diesen Verfahren oder durch Umestern des Dimethylesters nach der Methode von D.F. Faber et al [J. Org. Chem. 50. 3618 (1985)] hergestellt werden.

7,7-Ethylendioxy-cis-bicyclo[3.3.0]octan-3-on-2-carbonsäuremethylester (5)

Die Herstellung ist von verschiedenen Autoren beschrieben (vgl. l.c. Review von Nickolson et al).

7,7-(2,2-Dimethyltrimethylendioxy)-cis-bicyclo[3.3.0]octan-3-on-2-carbonsäuremethylester (6)

Die Herstellung erfolgt nach dem oben genannten Literaturverfahren aus 3,3-(2,2-Dimethyltrimethylendioxy)-cis-bicyclo[3.3.0]octan-7-on [E. Carceller et el, Tetrahedron Letters 25, 2019 (1984)].

cis-Bicyclo[3.3.0]octan-3,7-dion-2-carbonsäuremethylester (7)

Die Verbindung kann zum Beispiel durch Ketalspaltung von 5 bzw. 6 mit Säuren und Wasser oder auch durch Carboxylierung und Veresterung von cis-Bicyclo[3.3.0]octan-3,7-dion, zum Beispiel mit Magnesiummethylcarbonat [Reagenzherstellung: M. Stiles, H.L. Finkbeiner, J. Amer. Chem. Soc. 85. 616 (1963)] hergestellt werden.

Die durch die mikrobiologische Reduktion erhaltenen (1S,5S,6R,7R)-7-Hydroxy-bicyclo[3.3.0]octan-3-on-2,6-dicarbonsäurediester lassen sich nach herkommlichen chemischen Verfahren in Carbacycline überführen, zum Beispiel zu (1S,2R,3R,5R)-3-Hydroxy-7,7-ethylendioxy-bicyclo[3.3.0]-octan-2-carbonsäuremethylester, dessen Umsetzung in optisch aktives Carbacyclin inzwischen von Kojima et al. beschrieben wurde. Selbstverständlich lassen sich aus den nach dem erfindungsgemäßen Verfahren hergestellten Zwischenprodukten auch alle Carbacyclin-Analoga mit modifizierten Strukturen in der $\alpha$- und/oder $\omega$-Kette herstellen.

Die überzählige Carbonester-Gruppe wird als $\beta$-Ketoester nach an sich bekannten Verfahren decarbalkoxyliert. Besonders geeignet hierzu sind Umsetzungen mit 1,4-Diazabicyclo[2.2.2]octan [z.B. nach Miles et al, J. Org. Chem. 39, 2647 (1974)] oder mit Halogenid-Ionen in Dimethylsulfoxid oder Dimethylformamid [Krapcho, Synthesis 1982, 805, bzw. Mc Murray, Org. Reactions 24, 187 (1976)]. Man erhält so (1S,2R,3R,5R)-3-Hydroxy-bicyclo[3.3.0]octan-7-on-2-carbonsäureester.

Die erhaltenen (1S,2R,3R,5R)-3-Hydroxy-bicyclo[3.3.0]octan-7-on-2-carbonsäureester lassen sich durch übliche Verfahren in die Ketale überführen, indem sie mit 1,2- oder 1,3-Diolen unter Katalyse mit Säure oder sauren Salzen und Entfernen des Reaktionswassers durch Azeotrop-Destillation oder Wasser aufnehmende Mittel umgesetzt werden. Bei der Verwendung von Ethylenglykol erhält man so das von Kojima beschriebene Startmaterial für Carbacyclin.

**Beispiel 1**

cis-Bicyclo[3.3.0]octan-3,7-dion-2,6-dicarbonsäurediethylester

Man gibt 25 g cis-Bicyclo[3.3.0]octan-3,7-dion-2,4,6,8-tetracarbonsäure-tetraethylester zu einer auf 50 °C erwärmten Lösung von 14,1 g Natriumhydroxid in 68,5 ml Ethanol und 175 ml dimethylsulfoxid und rührt 18 Stunden bei dieser Temperatur unter Stickstoff. Nach dem Abkühlen gießt man auf 750 ml Eiswasser, säuert mit Essigsäure auf pH 5-6 an, extrahiert mit Ethylacetat, wäscht dieses mit Natriumhydrogencarbonat-Lösung und mit Natriumchlorid-Lösung, trocknet mit Natriumsulfat und konzentriert im Vakuum. Nach Umkristallisation aus Ethanol erhält man 13,37 g Produkt vom Schmelzpunkt 98 - 100 °C.

**Beispiel 2**

7,7-(2,2-Dimethyl-trimethylendioxy)-cis-bicyclo[3.3.0]octan-3-on-2-carbonsäuremethylester

Man suspendiert 38,34 g 55 - 60 %iges Natriumhydrid in 616 ml Dimethylcarbonat, erwärmt unter Stickstoff auf 50 °C und gibt eine kleine Menge der Lösung von 49,31 g 3,3-(2,2-Dimethyltrimethylendioxy)-cis-bicyclo-[3.3.0]octan-7-on in 370 ml Dimethylcarbonat dazu. Durch Zugabe von 2 ml Methanol induziert man die Reaktion, gibt die restliche Lösung dazu und rührt insgesamt 7,5 Stunden bei 50 °C. Man kühlt im Eisbad, zersetzt überschüssiges Natriumhydrid mit Methanol, gibt Wasser hinzu und neutralisiert mit Essigsäure. Man extrahiert das Produkt mit Dichlormethan, konzentriert im Vakuum und kristallisiert das Produkt mit Hexan. Man erhält 53,44 g Produkt vom Schmelzpunkt 72 °C.

**Beispiel 3**

cis-Bicyclo[3.3.0]octan-3,7-dion-2-carbonsäuremethylester

a) Man löst 15 g 7,7-(2,2-Dimethyltrimethylendioxy)-cis-bicyclo[3.,3,0]octan-3-on-2-carbonsäuremethylester in 350 ml Aceton, fügt 7,23 g Kaliumhydrogensulfat hinzu und erwärmt 1 Stunde auf 40 °C. Nach dem Abkühlen neutralisiert man mit i1n-Kaliumhydroxid-Lösung, destilliert das Aceton im Vakuum ab und extrahiert mit Dichlormethan. Man erhält 10,6 g Produkt, das aus Diisopropylether umkristallisiert werden kann (Schmelzpunkt 69 °C).

b) Man erhitz unter Stickstoff 26,0 g cis-Bicyclo[3.3.0]octan-3,7-dion mit 350 ml 2,15-molarer Magnesiummethylcarbonat-Lösung in Dimethylformamid für 4 Stunden, kühlt ab und gibt die Mischung langsam in 500 ml auf -65 bis -50 °C gekühlte 8,8-molare methanolische Salzsäure. Man läßt über Nacht auf 20 °C erwärmen, konzentriert im Vakuum, versetzt mit Wasser und extrahiert mit Diethylether. Nach Trocknung mit Natriumsulfat wird im Vakuum konzentriert und der erhaltene Rückstand an Kieselgel mit Ethylacetat/Methanol 95:5 chromatographiert. Man erhält 21,31 g Produkt (Schmelzpunkt 67 - 69 °C).

**Beispiel 4**

Eine 4 - 8 Tage alte Schrägagarkultur des Stammes Rhizopus oryzae (CBS 32 947) wird mit 3 ml physiologischer Kochsalzlösung abgeschwemmt und damit ein 2 l-Erlenmeyerkolben beimpft, der 500 ml sterile Nährlösung folgender Zusammensetzung enthält:

3,0 % Glucose
1,0 % Corn steep liquor
0,2 % $NaNO_3$
0,1 % $KH_2PO_4$
0,2 % $K_2HPO_4$
0,05 % $MgSO_4.7H_2O$
0,002 % $FeSO_4.7H_2O$
0,05 % KCl

Die Kultur wird 56 Stunden auf einem Rotationsschüttler bei 30 °C geschüttelt, wobei sie meist zu großen Klumpen auswächst. Zur besseren Beimpfbarkeit werden die Klumpen im Impfkolben unter sterilen Bedingungen mit einem Ultra-Turrax T 45 zellschonend zerkleinert.

Mit der turraxierten Pilzsuspension zweier Impfkolben wird ein 40 l-Stahlfermenter beimpft, der mit 28 l sterilisierter Nährlösung der gleichen Zusammensetzung wie die Vorkultur gefüllt ist. Gleichzeitig wird die Substratsuspension zugegeben (400 mg/l).

Substratsuspension: 12 g (±)-cis-3,7-Dioxo-bicyclo[3.3.0]octan-2,6-dicarbonsäure-dimethylester werden in 1200 ml autoklavierter 2 %iger Tween 80-Lösung (VE-Wasser) im Substratkolben mit einem Ultra-Turrax T45 homogenisiert.

Nach einer Kontaktzeit von 52 Stunden sind ca. 50% des eingesetzten racemischen Ketons umgesetzt. Die Kulturbrühe wird nun über Zellstoff-Gaze abgesaugt, das Filtrat 1x mit der Hälfte und anschließend noch 2x mit je 1/3 des Kulturvolumens mit MiBK extrahiert. Die Extrakte werden vereinigt und im Vakuum eingedampft, wobei die während der Fermentation gebildete Fumarsäure (1-1,5 g/l) auskristallisiert. Nach dem Abfiltrieren der Fumarsäure wird das Filtrat zur Trockne eingeengt und der Rückstand zur Abtrennung des unumgesetzten Ketons sowie zur Reinigung der 3α-OH-Verbindung über eine Kieselgelsäule mittels eines Lösungsmittelgradienten Hexan-Essigester chromatographiert. Man erhält 4,9 g (+)-cis-3α-Hydroxy-7-oxo-bicyclo[3.3.9]octan-2,6-dicarbonsäure-dimethylester vom Schmelzpunkt 54-56 °C mit einem Drehwert $[\alpha]^D$ + 51° (c = 1, Chloroform), was einer Enantiomerenreinheit von über 90 % ee entspricht.

**Beispiel 5**

Eine Schrägagarkultur des Stammes Rhodotorula spec. ATCC 18101 wird mit 3 ml physiologischer Kochsalzlösung abgeschwemmt und damit ein 2 l-Erlenmeyerkolben beimpft, der 500 ml sterile Nährlösung folgender Zusammensetzung enthält:

5 % Dextrose-Monohydrat
2 % Corn steep liquor
pH 6,5

Nach 2tägigem Schütteln auf einem Rotationsschüttler bei 30 °C wird mit 250 ml dieser Vorkultur ein 5 l-Fermenter beimpft, der mit 3,5 l sterilisierter Nährlösung der gleichen Zusammensetzung wie die Vorkultur gefüllt ist. Die Kultur wird 12 Stunden unter Belüftung (3,75 l/Min.) und Rühren (220 U/Min.) unter Konstanthaltung des pH bei 6,5 germiniert und sodann das Substrat in Form einer steril filtrierten Lösung von 1,5 g (±)-7,7-(2,2-Dimethyltrimethylendioxy)-cis-bicyclo[3.3.0]octan-3-on-2-carbonsäuremethylester in 50 ml Ethanol zugegeben. Nach einer Kontaktzeit von 12 Stunden sind 50 % des eingesetszten racemischen Ketons umgesetzt. Die Kulturbrühe wird nun 1x mit der Hälfte und anschließend noch 2x mit je 1/3 des Kulturvolumens mit Methylisobutylketon extrahiert. Die Extrakte werden vereinigt und im Vakuum zur Trockne eingedampft. Der verbliebene Rückstand wird zur Abtrennung des reduzierten (-)-Enantiomeren über eine Kieselgelsäule chromatographiert (Lösungsmittelgradient Dichlormethan-Dichlormethan / 8 % Aceton). Fraktion I enthält das natürlich konfigurierte unumgesetzte (+)-Ketoenantiomere. Nach dem Einengen zur Trockne und Kristallisation aus Hexan erhält man 580 mg (+)-7,7-(2,2-Dimethyltrimethylen-dioxy)-cis-bicyclo[3.3.0]octan-3-on-2-carbonsäuremethylester vom Schmelzpunkt 64-65 °C.

Zur chemischen Reduktion werden 500 mg (+)-7,7-(2,2-Dimethyltrimethylendioxy)-cis-bicyclo[3.3.0]-octan-3-on-2-carbonsäuremethylester in 10 ml Methanol gelöst und 1 Stunde am Rückfluß gekocht. Anschließend wird auf -40 °C abgekühlt, 200 mg $NaBH_4$ zugegeben und 1 Stunde bei dieser Temperatur gerührt. Danach gibt man 2 ml Aceton zu, rührt noch 30 Minuten nach, stellt mit verd. Essigsäure pH7 ein, verdünnt mit Wasser und extrahiert mit Dichlormethan. Der Extrakt wird zur Trockne eingeengt, der Rückstand in 10 ml Methanol gelöst, 100 mg Natriummethylat zugegeben und 4 Stunden bei 40 °C gerührt. Nun bringt man die Lösung mit $2nH_2SO_4$ unter Eiskühlung auf pH6,5, destilliert das Methanol ab,

nimmt den Rückstand in Wasser auf und extrahiert 3x mit Dichlormethan. Der Extrakt wird neutral gewaschen, über $Na_2SO_4$ getrocknet und zur Trockne eingeengt. Man erhält nach Kristallisation aus Hexan 420 mg (+)-7,7-(2,2-Dimethylthrimethylendioxy)-3α-hydroxy-cis-bicyclo[3.3.0]octan-2-carbonsäuremethylester vom Schmelzpunkt 59-61 °C und einem Drehwert von $[\alpha]_D$ +25,1° (c=1,2,Chloroform).

**Beispiel 6**

(1S,2R,3R,5R)-3-Hydroxy-cis-bicyclo[3.3.0]octan-7-on-2-carbonsäuremethylester

3,41 g des im Beispiel 4 erhaltenen Produkts erhitz man 1 Stunde unter Stickstoff mit 0,35 g Natriumchlorid und 41,2 ml Dimethylformamid, das 3% Wasser enthält. Man destilliert im Vakuum weitgehend ab und erhält 3,32 g des rohen Produkts, das in die Folgestufe eingesetzt wird.

Gereinigtes Produkt zeigt einen Schmelzpunkt von 55 °C und einen spezifischen Drehwert (c=1 in Chloroform) von $[\alpha]_D$ +6,0°.

**Beispiel 7**

(1S,2R,3R,5R)-7,7-Ethylendioxy-3-hydroxy-cis-bicycl[3.3.0]-octan-2-carbonsäuremethylester

Man erhitzt das Rohprodukt aus Beispiel 6 in 50 ml Benzol mit 1,13 g Ethylenglykol und 0,35 g 4-Toluolsulfonsäure für 3 Stunden zum Rückfluß und trennt entstehendes Wasser ab. Nach dem Abküglen versetzt man mit 0,5 g Natriumhydrogencarbonat, extrahiert mit Wasser und konzentriert im Vakuum. Man chromatographiert an Kieselgel mit Hexan-Ethylacetat-Gemischen und erhält 2,50 g Produkt als farbloses Öl. Spezifischer Drehwert (c=1 in Chloroform) $[\alpha]_D$ +26,5°.

**Patentansprüche**

**1.** Verfahren zur Herstellung von 3α-Hydroxy-prostacyclin-Zwwischenprodukten der Formel (+) -I

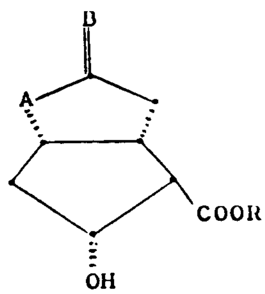

(+) - I,

worin

- A die Reste $-CH_2-$ oder >CH-COOR,
- B Sauerstoff oder den 2-bindigen Rest -O-X-O- mit X als geradkettigem oder verzweigtkettigem Alkylen mit 1 - 7 C-Atomen und
- R eine geradkettige oder verzweigtkettige Alkylgruppe mit 1 - 6 C-Atomen oder Benzyl bedeuten,

dadurch gekennzeichnet, daß man ein racemisches Bicyclooctandion der Formel (±) - II

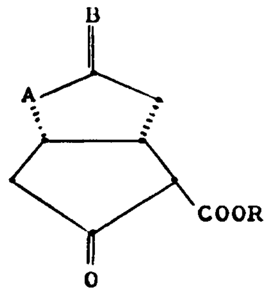

(±) - II

worin A, B und R die oben angegebene Bedeutung haben, mit Rhizopus-, oder Rhodotorula- Stämmen behandelt und für den Fall, daß der eingesetzte Stamm die (-)-Form aus dem Racemat (±) - II reduziert, eine chemische oder mikrobiologische Reduktion des unveränderten Bicyclooctandions der Formel (+) - II anschließt.

## Claims

**1.** Process for the preparation of 3α-hydroxyprostacycline intermediates of the formula (+) - I

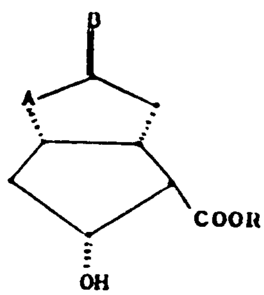

(+) - I

wherein

A represents a radical $-CH_2-$ or >CH-COOR,

B represents oxygen or the divalent radical -O-X-O-, wherein X represents straight-chained or branched-chained alkylene having from 1 to 7 carbon atoms, and

R represents a straight-chained or branched-chained alkyl group having from 1 to 6 carbon atoms, or represents benzyl,

characterised in that a racemic bicyclooctanedione of the formula (±) - II

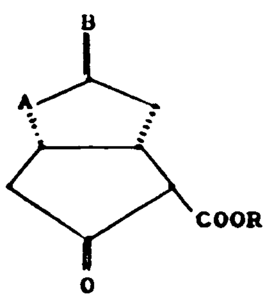

(±) - II,

wherein A, B and R have the meanings given above, is treated with Phizopus or Phodotorula strains and, in the case where the strain used reduces the (-) form of the racemate (±) - II, chemical or microbiological reduction of the unchanged bicyclooctanedione of the formula (+) - II is subsequently carried out.

## Revendications

1. Procédé pour préparer des corps intermédiaires pour prostacyclines, en l'espèce des composés hydroxy-3α répondant de formule (+)-I.

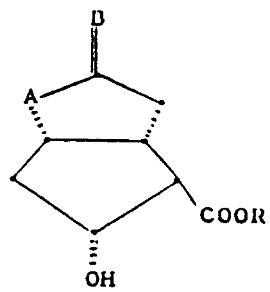

(+) - I.

dans laquelle

- A représente un radical $-CH_2$-ou >CH-COOR,
- B représente l'oxygène, ou un radical divalent -O-X-O- dans lequel X désigne un alkylène, linéaire ou ramifié, contenant de 1 à 7 atomes de carbone, et
- R représente un alkyle, linéaire ou ramifié, qui contient de 1 à 6 atomes de carbone, ou un radical benzyle,

procédé caractérisé en ce qu'on traite une bicyclo-octane-dione racémique répondant à la formule (±)-II :

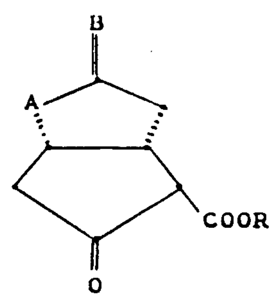

(±) - II

dans laquelle A, B et R ont les significations qui leur ont été données plus haut, par des souches de Rhizopus ou de Rhodotorula, et, dans le cas où la souche mise en jeu réduit la forme (-) du racémique (±)-II, on réduit ensuite, par voie chimique ou microbiologique, la bicyclo-octane-dione de formule (+)-II inaltérée.